# EUROPEAN PATENT SPECIFICATION

(11) **EP 1 928 412 B1**
(45) Date of publication and mention of the grant of the patent: **16.09.2015**
(21) Application number: 06842719.4
(22) Date of filing: 11.08.2006
(51) Int. Cl.: A61K 9/00, A61K 47/48, A61K 31/05, A61K 31/167

(54) **AQUEOUS ANAESTHETIC COMPOSITIONS COMPRISING PROPOFOL**
WÄSSRIGE NARKOSEZUSAMMENSETZUNGEN MIT PROPOFOL
COMPOSITIONS AQUEUSES ANESTHESIQUES COMPRENANT DU PROPOFOL

(30) Priority: 12.08.2005 IN MU09402005
(43) Date of publication of application: 11.06.2008
(73) Proprietor: Bharat Serums & Vaccines Ltd., 400 021 Mumbai (IN)
(72) Inventor: DAFTARY, Gautam, Vinod, Mumbai 400 021 (IN); PAI, Srikanth, Annappa, Mumbai 400 021 (IN); KULKARNI, Mangesh, Manikrao, Thane(w) 400 604 (IN)
(74) Representative: Beck Greener
(86) International application number: PCT/IN2006/000299
(87) International publication number: WO 2007/052295

(56) References cited:
- WO-A-96/32135
- WO-A-03/063824
- WO-A-2004/108113
- WO-A1-02/074200
- US-A1- 2004 014 718
- TRAPANI G ET AL: "INCLUSION COMPLEXATION OF PROPOFOL WITH 2-HYDROXYPROPYL-BETA- CYCLODIXTRIN. PHYSICOCHEMICAL, NUCLEAR MAGNETIC RESONANCE SPECTROSCOPIC STUDIES, AND ANESTHETIC PROPERTIES IN RAT" JOURNAL OF PHARMACEUTICAL SCIENCES, AMERICAN PHARMACEUTICAL ASSOCIATION. WASHINGTON, US, vol. 87, no. 4, April 1998 (1998-04), pages 514-518, XP000739683 ISSN: 0022-3549 cited in the application
- DOLLO GILLES ET AL: "Inclusion complexation of amide-type local anaesthetics with beta-cyclodextrin and its derivatives. I. Physicochemical characterization" INTERNATIONAL JOURNAL OF PHARMACEUTICS (AMSTERDAM), vol. 131, no. 2, 1996, pages 219-228, XP002431929 ISSN: 0378-5173

## Description

### Field of Invention

The present invention relates to aqueous anaesthetic compositions suitable for parenteral administration. In particular, it relates to aqueous solutions of Propofol complexed with 2-hydroxypropyl-β-cyclodextrin (referred to hereinafter as HPBCD).

### Background and prior art:

Propofol is an intravenous anaesthetic agent used for the induction and maintenance of anaesthesia as well as sedation. It has the desirable property of causing a rapid induction of and quick recovery from anaesthesia.

Despite being a preferred anaesthetic agent, the aqueous insolubility of Propofol has presented significant difficulties. It was first formulated as an aqueous solution containing polyethoxylated castor oil as a solubiliser, but this was found to be unacceptable due to anaphylactoid reactions in some patients. Subsequently, Propofol was reformulated as an oil in water emulsion using a mixture of soya oil and purified egg phosphatide. However, these lipid-based emulsions still suffered from several limitations. In particular, the formulation causes pain on injection in a significant number of patients, especially when injected into a small vain. The emulsions are also particularly susceptible to microbial growth such that strict aseptic techniques must be maintained even where antimicrobial preservatives are used. The emulsions also show poor physical stability, the potential for embolism, and increased fat load.

These difficulties, and in particular the tendency of the Propofol emulsion to cause pain on injection, have resulted in a number of attempts to provide improved aqueous Propofol formulations. It is believed that it is the presence of Propofol in the aqueous phase is responsible for the pain on injection. A number of attempts have therefore been made to alter the pharmacokinetics and pharmacodynamics of the formulations used so as to shift more of the Propofol into the non-aqueous phase of the emulsion.

The possibility of adding a local anaesthetic to the emulsion so as to reduce the perceived pain on injection has also been investigated. However, the addition of local anaesthetics or other electrolytes has been found to destabilize the Oil in water emulsion resulting in aggregation and coalescence of the oil phase. As such, premixed formulations containing oil in water Propofol emulsion and a local anaesthetic have proved to be pharmaceutically unacceptable. Where considered necessary, pre-treatment the area with a separate injection of local anaesthetic is therefore currently preferred.

As an alternative to using oil in water emulsions, several studies have investigated the possibility of using cyclodextrins or cyclodextrin derivatives to solubilise the Propofol. Cyclodextrins are cyclic compounds comprising a ring of six to eight glucopyranose units having a hydrophilic exterior surface and hydrophobic interior surface, and which act to solubilise hydrophobic compounds such as Propofol via inclusion complexation. Generally speaking, cyclodextrin derivatives are preferred due to the tendency of cyclodextrin to dissociate from the complexed hydrophobic compound on injection into the blood stream, as well as the tendency of cyclodextrin to interact preferably with cell membrane components.

One cyclodextrin derivative is 2-hydroxypropyl-β-cyclodextrin. G. Trapene at al (J. Pharm. Science, 1998, 84(4) (514 to 518)) discusses the physiochemical and anaesthetic properties of HPBCD : Propofol and indicates that a stable aqueous solution can be achieved with a 1:1 molar ratio of HPBCD:Propofol. International publication No. WO 96/32135 also discloses the use of Propofol : 2-hydroxypropyl-β-cyclodextrin complexes in which a Propofol to HPBCD molar ratio of 1 :1.5 to 1 :2.5 was shown to provide a stable aqueous solution of Propofol.

As described in Indian Patent No. 187686, the use of a propofol to HPBCD ratio from about 1:30 to about 1:60 results in an aqueous propofol formulation, which remains stable on autoclaving. There is, however, no provision for reduction in pain in this composition.

Another cyclodextrin derivative is sulfobutyl-ether-cyclodextrin (hereinafter referred to as SBECD). International publication No. WO 02/074200 discusses the use of SBECDs to solubilise Propofol. It also makes general reference to a list of local anaesthetics to be mixed with SBECD: propofol solutions, although some of those suggested (eg. benzocaine, tetracaine) appear surprising in that they would result in systemic toxicity if intravenously injected.

However, the clinical usage of SBECD is yet to be established as safe, as no products containing SBECD are available commercially.

WO2004/108113A1 discloses a ready to use anaesthetic composition comprising propofol, at least one analgesic, and antioxidants in a solvent system. The solvent system may be selected from (a) water and 2,5 di-omethyl-1,4,3,6-dianhydro-D-glucitol and/or its derivatives, and (b) cyclodextrin and its derivative and water wherein cyclodextrin forms a complex with Propofol. In certain embodiments, cyclodextrin and propofol may present in a ratio of 1:3, preferably 1:1. In certain embodiments, the analgesic may be selected from Lignocaine, Tramadol, Ketorolac, Ketamine, Alfentanil, Pethidine, Bupivacaine, Ropivacaine and other analgesics that may be administered parenterally. In all the exemplified embodiments, the composition comprises propofol in a solvent system that is a 65:30 solution of 2,5 di-omethyl-1,4,3,6-dianhydro-D-glucitol in water, together with an analgesic selected from lignocaine or Ketamine.

Thus there is a long standing need to have a stable single injectable composition of Propofol with a safe pain reducing local anaesthetic to facilitate easy one shot administration.

### Object

The main object of the present invention is to provide a stable aqueous anaesthetic composition suitable for parenteral administration comprising propofol, 2-hydroxypropyl-β-cyclodextrin (HPBCD) and a safe local anaesthetic.

According to further aspect of the object of the present invention is to provide a suitable method for manufacturing an aqueous anaesthetic composition, suitable for parenteral administration, comprising Propofol, 2-hydroxypropyl-β-cyclodextrin (HPBCD), and a safe local anaesthetic.

### Summary of the Invention

The present invention provides an aqueous anaesthetic composition suitable for parenteral administration comprising propofol, 2-hydroxypropyl-β-cyclodextrin (HPBCD) and a local anesthetic Lignocaine or its acid salts, the weight ratio of propofol to HPBCD being 1:14 to 1:60.

The present invention also provides a method of manufacturing an aqueous anaesthetic composition suitable for parenteral administration as recited above, comprising forming an aqueous solution of propofol, 2-hydroxypropyl-β-cyclodextrin (HPBCD) and a local anaesthetic Lignocaine or its acid salts at pH 4-7 using buffers, and/or acids like hydrochloric acid, or phosphoric acids, and/or alkali like sodium hydroxide.

### Detailed description of the Invention

Presently, Propofol Injection and Lignocaine Injection are available separately. They are mixed just prior to administration. As the existing emulsion product is unstable on keeping after mixing with Lignocaine. The present invention now offers for the first time a single injection giving Propofol together with local anesthetic, Lignocaine to reduce pain on injection of Propofol.

Local anaesthetics inhibit the conduction of sensory nerve impulses by permeating across the nerve cell membrane and reversibly complexing with the intra-cellular side of the sodium Ion pump, thereby decreasing the permeability of the nerve cell to sodium ions and thus inhibiting the transmission of nerve impulses. The chemical structure of a local anaesthetic comprises three groups, a lipophilic group (usually comprising a benzene ring) that allows the compound to permeate across the nerve cell membrane, an intermediate chain (usually comprising an ester or amide linkage), and an ionisable group (normally a tertiary amine) that allows the anaesthetic to be solubilised in the aqueous environment inside and outside the nerve cell.

The local anaesthetic may be used in its base form. It may react with phenolic ( i.e. acidic) Propofol and hence acidic pH is useful in preventing such reaction and keeping the base free for reducing pain. The Lignocaine base may be used as an aqueous solution or in an acidic solution or as its water-soluble salts. It can also be used as a solution in carbonated base. Use of the local anaesthetic, Lignocaine in the form of its hydrochloride salt is generally preferred.

With this thinking, we have now found that Propofol, HPBCD and Lignocaine or its acid salts can be made to give stable aqueous compositions in acidic pH. An acidic pH additionally helps to control microbial growth during long term administration of the composition.

Preferably the pH of the composition is below 7, more preferably in the range of 4-7 and most preferably in the range 4.5 to 6.5.

The composition further comprises, acidifying agents and/or alkalising agents and/or antioxidants and/or buffers.

The antioxidant is selected from EDTA or a salt thereof, sodium metabisulphite, acetylcysteine, or ascorbic acid. Preferably the antioxidant is disodium edetate.

The compositions of the present invention may contain pharmaceutically acceptable acidifying agents and / or alkalizing agents and / or buffers for adjusting and stabilizing the pH of the solutions. Acidifying agents may include inorganic acids and / or organic acids and / or inorganic salts and / or organic salts. Alkalizing agents may include inorganic bases and / or organic bases and / or inorganic salts and / or organic salts. Examples of acidifying agents may be but are not limited to hydrochloric acid, carbonic acid, phosphoric acid, histidine HCl, glycine HCl, citric acid. Examples of alkalizing agents may be but are not limited to sodium hydroxide, potassium hydroxide, ammonium hydroxide, tromethamine, histidine.

Use of buffer helps in maintaining pH and increasing stability of the composition. The buffer may be selected from any pharmaceutically acceptable buffer systems such as, citrate buffer, phosphate buffer, histidine or glycine buffer containing any of the commonly used compounds, or a mixture of compounds such as citric acid, sodium citrate, potassium citrate, glycine, histidine, histidine HCl, phosphoric acid sodium phosphate, disodium hydrogen phosphate, sodium dihydrogen phosphate, potassium phosphate, dipotassium hydrogen phosphate. potassium dihydrogen phosphate.

The isotonic diluent may be selected from pharmaceutically acceptable diluents such as dextrose, sodium chloride and mannitol. However, glycerol or other polyols are not preferred.

Not bound by theory we believe that polyols such as glycerin, propylene glycol, or polyethylene glycol of a low molecular weight, act as co-solvents for propofol and increase free propofol in aqueous phase and increase pain on injection. Therefore use of such polyols is not preferred in these compositions.

The composition has a Propofol:HPBCD weight ratio of 1:14 - 1:60. The weight ratio of propofol to HPBCD is more preferably 1:14 - 1:30 and most preferably 1:20 - 1:30.

The composition further comprises an antioxidant, a buffer, an isotonic diluent or a combination thereof.

Preferably, the propofol content of the solution in the present invention is 1mg/ml - 20mg/ml and more preferably 1 mg/ml - 10 mg/ml, most preferably about 10 mg/ml.

The local anaesthetic used in the composition of the present invention is Lignocaine base and/or its acid salts. Its content in the composition expressed *as* Lignocaine base is 0.5 mg/ml - 1.5 mg/ml. More preferably the content of Lignocaine in the composition of present invention is about 1mg/ml.

We have now found that aqueous compositions of the present invention comprising Propofol to HPBCD in the ratio of 1:14 - 1:60 and Lignocaine or its acid salts expressed as Lignocaine base 0.5 - 1.5mg/ml of the composition at pH 4 - 7 are stable on autoclaving.

The composition of the present invention is manufactured by
1. *Dissolving HPBCD in required quantity of water or in a suitable buffer.
2. *Lignocaine as such or as a stock solution is added into HPBCD solution and dissolved by stirring and if required pH of the solution is adjusted between 4.5 - 7.0 with acidifying agents / alkalizing agents.
3. Propofol is added and dissolved by stirring under nitrogen cover.
4. The pharmaceutically acceptable additives like antioxidants, tonicity agents, as such or as stock solutions are added into Propofol -Lignocaine - HPBCD solution and mixed by stirring.
5. The resultant is then diluted to required volume and filtered through 0.45 and / or 0.22µ filter.
6. The filtered solution is then filled into glass containers, head space of the containers are purged with Nitrogen gas before sealing and sterilized by autoclaving.
*Steps 1, and 2 may be done in alternative ways as follows:
Alternatively Lignocaine can be added as such or as a stock solution in acidic pH after complexing Propofol with HPBCD
   OR
Lignocaine can be added into water acidic pH before the addition of HPBCD.

Thus in general, the method of manufacturing an aqueous anaesthetic \ composition suitable for parenteral administration, comprises forming an aqueous solution of propofol, 2-hydroxypropyl-β-cyclodextrin (HPBCD) and a local anaesthetic Lignocaine or its acid salts at pH 4-7 using buffers, and/or acids like hydrochloric acid or phosphoric acids, and/or alkali like sodium hydroxide.

### Examples

The invention will now be illustrated by way of Examples. The Examples are by way of illustration only and in no way restrict the scope of the invention.

### Materials and equipment used in the Examples:

Propofol complies with the European Pharmacopoeia (Ph.Eur.) specifications,
Lignocaine hydrochloride (Lidocaine hydrochloride) complying with Indian Pharmacopoeia (I.P.) specifications is used.

Lignocaine (Lidocaine) complying with United States Pharmacopoeia (USP) specifications is used.

2-hydroxypropyl-β-cyclodextrin (HPBCD), Pharma grade is manufactured by M/s. Wacker Chemie.

### Example I:

1 % Propofol Lipid Free with 0.1 % Lignocaine (as HCl)

The following composition was prepared by the procedure given below:

| **Ingredients** | **Qty** |
|---|---|
| a) Propofol | 1g |
| b) 2-hydroxypropyl-β-cyclodextrin | 30g |
| c) Lignocaine HCl eq. to base | 0.1g |
| d) Disodium edetate | 0.0059g |
| e) Water for Injection | q.s.to 100ml |

### Procedure:

2-hydroxypropyl-β-cyclodextrin (30g) was dissolved in 55ml of Water for Injection. Lignocaine HCl (0.1234 g) was added into HPBCD solution and dissolved by stirring. Propofol was then added to HPBCD solution slowly under stirring. This solution was stirred at moderate speed for 3 hours to bring about complexation of Propofol with HPBCD.

Disodium edetate solution in water was added to the above solution under stirring. The volume was made upto 100ml with water. The clear solution obtained was filtered through 0.2µ filter, filled into glass vials under nitrogen, sealed and autoclaved.

The composition had a pH of 5.82

### Example II:

Preclinical evaluation of Anesthetic Activity of composition of Example I. Induction and recovery time after administering anesthetic composition of Example I were evaluated in comparison with commercially available Propofol Oil-in-Water emulsion in Swiss albino mice.

The results are as given in Table 1:

**Table 1**

| **Dose of Propofol (mg/Kg body weight)** | **Induction time (seconds)** | | **Recovery time (minutes)** | |
|---|---|---|---|---|
| | Example I | Propofol o / w emulsion | Example I | Propofol o / w emulsion |
| 25 | 9.60 ± 0.97 | 9.90 ± 0.31 | 5.27 ± 2.23 | 4.29 ± 0.89 |

The results show that the Induction and recovery time of composition of Example I was comparable with that of Propofol Oil-in-Water emulsion indicating similar pharmacokinetic and pharmacodynamic profile of two pharmaceutically different compositions.

### Example III: Acute toxicity of composition of Example I in mice

The composition obtained in Example I was subjected to acute toxicity studies in mice. The composition of Example I was suitably diluted with 5% Dextrose Injection and administered intravenously. Propofol in the doses of 30 mg/kg, 35 mg/kg and 40 mg/kg body weight was administered in three different groups of animals, each group consisting of ten animals.

The animals were kept under observation for 14 days and mortality recorded at the end of 3 days and 7 days.

The mortality observed at different doses is provided in Table 2:

**Table 2**

| **Composition of Example I** | | |
|---|---|---|
| **Dose (mg/Kg body weight)** | **Mortality (%)** | |
| | **3 days** | **7days** |
| 35 | 20% | 20% |
| 40 | 60% | 60% |
| 45 | 80% | 80% |
| LD₅₀ | 39.23 | 39.23 |

### Example IV: Stability studies of composition of Example I

The composition obtained in Example I was subjected to stability studies at 25°C. The stability data is provided in Table 3:

**Table3**

| **Storage condition: 25°C** | | | | |
|---|---|---|---|---|
| | **Condition** | | | |
| | | **25°C** | | |
| **Test parameters** | **Initial** | **1 month** | **2 month** | **3 month** |
| Appearance | Clear, colourless solution | Clear, colourless solution | Clear, colourless solution | Clear, colourless solution |
| pH | 5.82 | 5.80 | 5.78 | 5.80 |
| *Assay of Propofol (%) | 100.1 | 99.0 | 99.80 | 98.50 |
| *Degradation products | | | | |
| Quinono Propofol | 0.03 | Nil | Nil | Nil |
| Propofol Dimer | 0.07 | 0.08 | 0.14 | 0.08 |
| *Assay of Lignocaine HCl as Lignocaine (%) | 100.4 | 100.8 | 101.0 | 100.0 |

| | | | | |
|---|---|---|---|---|
| * Methods are given at the end of all Examples. | | | | |

From the above it is evident that Propofol and Lignocaine (as HCl) are stable in the composition obtained in Example I without undergoing any degradation when stored at 25°C.

### Example V: 1% Propofol Lipid Free with 0.1% Lignocaine (as base)

The following composition was prepared by the procedure given below

| **Ingredients** | | **Qty** |
|---|---|---|
| a) | Propofol | 1g |
| b) | 2-hydroxyporpyl-β-cyclodextrin | 30g |
| c) | Lignocaine base | 0.1g |
| d) | Disodium edetate | 0.005g |
| e) | Hydrochloric acid (0.1 N) | 2.0ml |
| f) | Water For Injection | q.s.to 100ml |

### Procedure:

2-hydroxypropyl-β-cyclodextrin was dissolved in 55ml of Water for Injection. Lignocaine base was added into HPBCD solution and dissolved by stirring. The pH of the solution was adjusted to about 6.7 with 0.1 N Hydrochloric acid. Propofol was then added to HPBCD solution slowly under stirring. This solution was stirred at moderate speed for 3 hours to bring about complexation of Propofol with HPBCD.

Disodium edetate was added to the above solution under, the volume was made upto 100ml with water. The clear solution obtained was filtered through 0.2µ filter filled into glass vials under nitrogen, sealed and autoclaved.

The composition had a pH of 6.00.

### Preclinical evaluation of Anesthetic Activity of composition of Example V

Induction and recovery time after administering anesthetic composition of Example V were evaluated in comparison with Propofol Oil-in-Water emulsion in Swiss albino mice.

The results are provided in Table 4:

**Table 4**

| **Dose of Propofol (mg/Kg body weight)** | **Induction time (seconds)** | | **Recovery time (minutes)** | |
|---|---|---|---|---|
| | Example V | Propofol o / w emulsion | Example V | Propofol o / w emulsion |
| 25 | 9.90 ± 0.32 | 9.90 ± 0.31 | 4.61 ± 1.41 | 4.29 ± 0.89 |

The Induction and recovery time of composition of Example V was comparable with that of Propofol Oil-in-Water emulsion indicating similar pharmacokinetic and pharmacodynamic profile of two pharmaceutically different compositions

The composition of Example V was subjected for stability studies at 25°C. The data at the end of 3 months is provided in Table 5:

**Table 5**

| | **Condition** | | | |
|---|---|---|---|---|
| | | **25°C** | | |
| **Test parameters** | **Initial** | **1 month** | **2 month** | **3 month** |
| Appearance | Clear, colourless solution | Clear, colourless solution | Clear, colourless solution | Clear, colourless solution |
| pH | 6.60 | 6.60 | 6.55 | 6.54 |
| *Assay of Propofol (%) | 97.50 | 97.58 | 97.20 | 97.80 |
| *Degradation products | | | | |
| ▪ Quinono Propofol | 0.03 | Nil | Nil | Nil |
| ▪ Propofol Dimer | 0.06 | 0.07 | 0.01 | 0.053 |
| *Assay of Lignocaine (%) | 101.2 | 101.8 | 102.0 | 100.9 |

| | | | | |
|---|---|---|---|---|
| * Methods are given at the end of all Examples. | | | | |

From the above data it is evident that Propofol and Lignocaine (as base) are stable in the composition obtained in Example V without. undergoing any significant degradation when stored at 25°C.

Other compositions of the present invention are represented in Table 6, Table 7 and Table 8.

**Table 6**

| **Ingredients** | **Example VI** |
|---|---|
| Propofol | 1.0g |
| Lignocaine base | 0.1g |
| HPBCD | 30.0g |
| Disodium edentate | 0.0055g |
| Disodium Hydrogen phosphate | 0.01g |
| Potassium dihydrogen phosphate | 0.0425g |
| Phosphoric acid 0.5% | 4ml |
| Water for Injection | q.s 100ml |
| pH | 6.50 |

**Table 7**

| **Ingredients** | **Example VII** | **Example VIII** | **Example IX** |
|---|---|---|---|
| Propofol | 1.0g | 1.0g | 1.0g |
| Lignocaine base | ------- | 0.1g | **-**---- |
| Lignocaine HCl eq. to base | 0.1g | ----- | 0.1g |
| HPBCD | 22.0g | 22.0g | 14.0g |
| Disodium edetate | 0.0055g | 0.0055g | 0.0055g |
| Hydrochloric acid 0.1N | ------ | 4.0ml | ----- |
| Dextrose | ------ | ----- | 2.0 g |
| Water for injection | q.s.100ml | q.s.100ml | q.s.100ml |
| pH | 6.0 | 5.94 | 6.0 |

**Table 8**

| **Ingredients** | **Example X** | **Example XI** | **Example XII** | **Example XIII** |
|---|---|---|---|---|
| Propofol | 1.0g | 1.0g | 1.0g | 1.0g |
| Lignocaine base | ------ | 0.1g | ------ | 0.1g |
| Lignocaine HCl eq.to base | 0.1g | ------ | 0.1g | ------ |
| HPBCD | 14.0g | 14.0g | 30.0g | 30.0g |
| Disodium edetate | 0.0055g | 0.0055g | ------ | ------ |
| Hydrochloric acid 0.1N | ------ | 4.0ml | ------ | 4.2ml |
| Water for injection | q.s.100ml | q.s.100ml | q.s.100ml | q.s.100ml |
| pH | 6.0 | 5.94 | 5.98 | 6.02 |

The compositions shown in Table 6, Table 7 and Table 8 were prepared by the procedure given below.

Example VI: The compositions were prepared by following the procedure of Example V using the components in the amounts mentioned in Table 6. Buffering salts and phosphoric acid whenever present were added as aqueous solution to HPBCD solution before adding lignocaine.

Example VIII, XI & XIII: The compositions were prepared by following the procedure of Example V using the components in the amounts mentioned in Table 6 and Table 7.

Example VII, X & XII: The compositions were prepared by following the procedure of Example I using the components in the amounts mentioned in Table 6 and Table 7.

Example IX: The composition was prepared by following the procedure of Example I adding Dextrose at the end before filtration.

### Method for determination of Propofol content, Degradation products and Lignocaine content:

| 1. | **Propofol and degradation products content:** Propofol and degradation products content was determined by HPLC. The details are as follows: | |
|---|---|---|
| | Column | - Hypersil ODS |
| | Detector | - Ultraviolet detector |
| | Detection wavelength | - 270nm |
| | Mobile phase | - 60 : 15 : 25 Acetonitrile : methanol: 10mM |
| | | potassium phosphate Buffer |
| | Sample concentration | - 0.2mg/ml |
| | Flow rate | - 1ml / min. |
| | | |

| 2. | **Lignocaine content:** | |
|---|---|---|
| | Column | - Hypersil ODS |
| | Detector | - Ultraviolet detector |
| | Detection wavelength | - 235nm |
| | Mobile phase | - 60 : 15 : 25 Acetonitrile : methanol: 10mM |
| | | potassium phosphate Buffer |
| | Sample concentration | - 0.02mg/ml |
| | Flow rate | - 1ml / min. |

### Advantages of the present invention :

The present invention provides a clear sterile anaesthetic composition that overcomes the disadvantages of emulsion formulation. The composition of the present invention has many advantages some of which are as follows:
1. The composition causes less pain on injection.
2. The composition is clear, can be visually inspected before administration and can be administered with the use of on-line microbial filter.
3. The composition does not contain phospholipids. Hence plasma phospholipids are unaffected on parenteral administration of the composition.
4. The composition does not cause any change in triglyceride clearance.
5. The composition can be mixed with any of the commonly used diluents before administration.
6. The acidic pH of the composition helps to control microbial growth during long term administration.

## Claims

1. An aqueous anaesthetic composition suitable for parenteral administration comprising propofol, 2-hydroxypropyl-β-cyclodextrin (HPBCD) and a local anesthetic Lignocaine or its acid salts, the weight ratio of propofol to HPBCD being 1:14 to 1:60.

2. A composition according to Claim 1, wherein the Lignocaine acid salt is Lignocaine hydrochloride.

3. A composition as claimed in Claim 1 or 2, wherein the said composition has pH below 7.

4. A composition as claimed in Claim 3, wherein the said composition has pH 4 - 7.

5. A composition as claimed in any of the preceding claims, further comprising an antioxidant, a buffer, an isotonic diluent or a combination thereof.

6. A composition as claimed in Claim 5, wherein the antioxidant is EDTA or a salt thereof.

7. A composition as claimed in any of the preceding claims, wherein the weight ratio of propofol to HPBCD is 1:14 - 1:30.

8. A composition as claimed in Claim 7, wherein the weight ratio of propofol to HPBCD is 1:20 - 1:30.

9. A composition as claimed in any of the preceding claim, wherein the propofol content of the composition is 1 mg/ml - 20 mg/ml.

10. A composition as claimed in Claim 9, wherein the propofol content of the composition is about 10 mg/ml.

11. A composition as claimed in any of the preceding claim, wherein the Lignocaine or its acid salts content of the composition expressed as Lignocaine base is 0.5 mg/ml - 1.5 mg/ml.

12. A composition as claimed in Claim 11, wherein the Lignocaine or its acid salts content of the composition expressed as Lignocaine base is about 1 mg/ml.

13. A composition as claimed in any of the preceding claim, wherein Propofol is about 1g; 2-hydroxypropyl-β-cyclodextrin is about 30g; Lignocain HCl eq. to base, is about 0.1g; Disodium edetate is about 0.006g and Water for Injection q.s. to 100 ml and has a pH 4 - 7.

14. A composition as claimed in any of the preceding claim, wherein Propofol is about 1g; 2-hydroxypropyl-β-cyclodextrin is about 30g; Lignocaine base is about 0.1g; Disodium edetate is about 0.005g; Hydrochloric acid 0.1N is about 2 ml and Water for Injection q.s. to 100 ml and has a pH 4-7.

15. A method of manufacturing an aqueous anaesthetic composition suitable for parenteral administration as claimed in any of the preceding claim, comprising forming an aqueous solution of propofol, 2-hydroxypropyl-β- cyclodextrin (HPBCD) and a local anaesthetic Lignocaine or its acid salts at pH 4-7 using buffers, and/or acids like hydrochloric acid, or phosphoric acids, and/or alkali like sodium hydroxide.

## Patentansprüche

1. Wässrige anästhetische Zusammensetzung, geeignet zur parenteralen Anwendung, Propofol, 2-Hydroxypropyl-β-cyclodextrin (HPBCD) und ein Lokalanästhetikum Lignocain oder dessen saure Salze umfasst, wobei das Gewichtsverhältnis des Propofol zu dem HPBCD zwischen 1:14 und 1:60 beträgt.

2. Zusammensetzung nach Anspruch 1, wobei das saure Salz des Lignocain Lignocain-Hydrochlorid ist.

3. Zusammensetzung nach Anspruch 1 oder 2, wobei die Zusammensetzung einen pH-Wert unter 7 aufweist.

4. Zusammensetzung nach Anspruch 3, wobei die Zusammensetzung einen pH-Wert von 4-7 aufweist.

5. Zusammensetzung nach einem der vorstehenden Ansprüche, ferner ein Antioxidationsmittel, einen Puffer, ein isotonisches Verdünnungsmittel oder eine Kombination daraus umfasst.

6. Zusammensetzung nach Anspruch 5, wobei das Antioxidationsmittel EDTA oder ein Salz davon ist.

7. Zusammensetzung nach einem der vorstehenden Ansprüche, wobei das Gewichtsverhältnis des Propofol zu HPBCD zwischen 1:14 und 1:30 liegt.

8. Zusammensetzung nach Anspruch 7, wobei das Gewichtsverhältnis des Propofol zu dem HPBCD zwischen 1:20 und 1:30 beträgt.

9. Zusammensetzung nach einem der vorstehenden Ansprüche, wobei der Propofolgehalt der Zusammensetzung zwischen 1 mg/ml und 20 mg/ml beträgt.

10. Zusammensetzung nach Anspruch 9, wobei der Propofolgehalt der Zusammensetzung in etwa 10 mg/ml beträgt.

11. Zusammensetzung nach einem der vorstehenden Ansprüche, wobei der Gehalt des Lignocain oder seiner sauren Salze in der als Lignocain-Base angegebenen Zusammensetzung zwischen 0,5 mg/ml und 1,5 mg/ml beträgt.

12. Zusammensetzung nach Anspruch 11, wobei der Gehalt des Lignocain oder seiner sauren Salze in der als Lignocain-Base angegebenen Zusammensetzung in etwa 1 mg/ml beträgt.

13. Zusammensetzung nach einem der vorstehenden Ansprüche, wobei die Menge des Propofol etwa 1 g; des 2-Hydroxypropyl-β-cyclodextrin etwa 30 g; des Lignocain-HCl äquivalent zur Lignocain-Base etwa 0,1 g; des Dinatriumedetat etwa 0,006 g und des Wassers für die Injektion qs bis 100 ml beträgt und einen pH-Wert von 4-7 aufweist.

14. Zusammensetzung nach einem der vorstehenden Ansprüche, wobei die Menge des Propofol etwa 1 g; des 2-Hydroxypropyl-β-cyclodextrin etwa 30 g; der Lignocain-Base etwa 0,1 g; des Dinatriumedetat etwa 0,005 g; der 0,1 N-Salzsäure etwa 2 ml und des Wassers für die Injektion qs bis 100 ml beträgt und einen pH-Wert von 4-7 aufweist.

15. Verfahren zum Herstellen einer wässrigen anästhetischen Zusammensetzung, geeignet zur parenteralen Anwendung nach einem der vorstehenden Ansprüche, umfassend das Bilden einer wässrigen Lösung aus Propofol, 2-Hydroxypropyl-β-cyclodextrin (HPBCD) und einem Lokalanästhetikum Lignocain oder dessen saurer Salze bei einem pH-Wert von 4-7 unter Verwendung von Puffern und/oder aus Säuren wie Salzsäure oder Phosphorsäuren und/oder alkaliähnlichem Natriumhydroxid.

## Revendications

1. Composition anesthésique aqueuse pour une utilisation par administration parentérale comprenant du propofol, 2-hydroxypropyl-β-cyclodextrine (HPBCD) et un anesthésique local Lignocaïne ou ses sels d'acide, le ratio de poids du propofol sur HPBCD étant compris entre 1:14 et 1:60.

2. Une composition selon la Revendication 1, où le sel d'acide de Lignocaïne est l'hypochlorure de Lignocaïne.

3. Une composition selon la Revendication 1 ou 2, où ladite composition présente un pH inférieur à 7.

4. Une composition selon la Revendication 3, où ladite composition présente un pH compris entre 4 et 7.

5. Une composition selon l'une quelconque des revendications précédentes, comprenant également un antioxydant, une solution tampon, un diluant isotonique ou une combinaison de ceux-ci.

6. Une composition selon la Revendication 5, où l'antioxydant est l'EDTA ou un sel de ce dernier.

7. Une composition selon l'une quelconque des revendications précédentes, où le ratio de poids du propofol sur HPBCD est compris entre 1:14 et 1:30.

8. Une composition selon la Revendication 7, où le ratio de poids du propofol sur HPBCD est compris entre 1:20 et 1:30.

9. Une composition selon l'une quelconque des revendications précédentes, où la concentration en propofol de la composition est comprise entre 1 mg/ml et 20 mg/ml.

10. Une composition selon la Revendication 9, où la concentration en propofol de la composition est d'environ 10 mg/ml.

11. Une composition selon l'une quelconque des revendications précédentes, où la concentration en Lignocaïne ou ses sels d'acide de la composition exprimée sous forme de base de Lignocaïne est comprise entre 0,5 mg/ml et 1,5 mg/ml.

12. Une composition selon la Revendication 11, où la concentration en Lignocaïne ou ses sels d'acide de la composition exprimée sous forme de base de Lignocaïne est d'environ 1 mg/ml.

13. Une composition selon l'une quelconque des revendications précédentes, où la concentration en Propofol est d'environ 1 g ; 2-hydroxypropyl-β-cyclodextrine environ 30 g ; HCl de Lignocaïne éq. à la base, environ 0,1 g ; édétate de disodium environ 0,006 g et Eau pour Injection sous-cutanée sur 100 ml et présente un pH compris entre 4 et 7.

14. Une composition selon l'une quelconque des revendications précédentes, où la concentration en Propofol est d'environ 1 g ; 2-hydroxypropyl-β-cyclodextrine environ 30 g ; base de Lignocaïne environ 0,1 g ; édétate de disodium environ 0,005 g ; acide hydrochlorique 0,1N environ 2 ml et Eau pour Injection sous-cutanée sur 100 ml et présente un pH compris entre 4 et 7.

15. Un procédé de fabrication d'une composition anesthésique aqueuse pour une utilisation par administration parentérale selon l'une quelconque des revendications précédentes, formant une solution aqueuse de propofol, 2-hydroxypropyl-β-cyclodextrine (HPBCD) et un anesthésiant local Lignocaïne ou ses sels d'acide à un pH compris entre 4 et 7 utilisant des solutions tampons et/ou des acides tels que l'acide hydrochlorique, ou des acides phosphoriques, et/ou des bases telles que l'hydroxyde de sodium.
